(19) Europäisches Patentamt
European Patent Office
Office européen des brevets

(11) **EP 2 889 791 A1**

(12) **EUROPEAN PATENT APPLICATION**
published in accordance with Art. 153(4) EPC

(43) Date of publication:
**01.07.2015 Bulletin 2015/27**

(51) Int Cl.:
**G06F 19/16** (2011.01)   **C07B 61/00** (2006.01)
**C40B 30/02** (2006.01)   **G01N 33/15** (2006.01)
**G01N 33/50** (2006.01)

(21) Application number: **13832325.8**

(22) Date of filing: **24.08.2013**

(86) International application number:
**PCT/JP2013/072630**

(87) International publication number:
**WO 2014/034577 (06.03.2014 Gazette 2014/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **27.08.2012   JP 2012186072**

(71) Applicants:
• **Kyoto Constella Technologies Co. Ltd.**
**Kyoto 604-8156 (JP)**
• **Kyoto University**
**Sakyo-ku**
**Kyoto-shi**
**Kyoto 606-8501 (JP)**

(72) Inventors:
• **OKUNO, Yasushi**
**Kyoto-shi**
**Kyoto 606-8501 (JP)**
• **KANAI, Chisato**
**Kyoto-shi**
**Kyoto 604-8156 (JP)**
• **YOSHIKAWA, Tatsuya**
**Kyoto-shi**
**Kyoto 604-8156 (JP)**
• **TAMON, Akiko**
**Kyoto-shi**
**Kyoto 604-8156 (JP)**

(74) Representative: **Schiweck, Weinzierl & Koch**
**European Patent Attorneys**
**Landsberger Straße 98**
**80339 München (DE)**

(54) **COMPOUND DESIGN DEVICE, COMPOUND DESIGN METHOD, AND COMPUTER PROGRAM**

(57) When the interaction of a compound is predicted by using a computer, a technique to highly precisely design a compound having a novel structure has been required. A compound designing device is provided which includes an input unit configured to receive, at least about one or more query proteins, one or more pieces of query protein information corresponding to the one or more query proteins; and a processing unit configured to perform steps of (a) generating one or more pieces of compound information, (b) computing a score indicating interaction potential between a compound corresponding to the compound information and each of the one or more query proteins, (c) updating the compound information by an optimization method with reference to the score computed at step (b) such that the interaction potential increases, and (d) repeating steps (b) and (c) a plurality of times.

Figure 2

EP 2 889 791 A1

**Description**

TECHNICAL FIELD

The present invention relates to compound designing devices for designing compounds which interact with proteins, compound designing methods for designing compounds which interact with proteins by using computers, and computer programs configured to allow computers to design compounds which interact with proteins.

BACKGROUND ART

Examples of an approach to predict the interaction between a protein, at which development of drugs is targeted, and a compound include an approach in which by using the spatial configuration information of the protein experimentally obtained by, for example, NMR or an X-ray crystal structure analysis, a binding site to the compound is evaluated by docking with the compound (see, for example Patent Documents 1-3). Moreover, an approach to design a compound having a novel structure by a computer is de novo design. Examples of the de novo design using particle swarm optimization as an optimization method include the technology described in, for example, Non-Patent Document 1.
In recent years, a technique for predicting the interaction between a protein and a compound from the amino acid sequence information of the protein, various descriptors of the compound, etc. by using a pattern recognition technology such as a support vector machine has been developed (see Patent Documents 4 and 5).

CITATION LIST

PATENT DOCUMENT

Patent Document 1: Japanese Unexamined Patent Publication No. 2009-007302

Patent Document 2: Japanese Unexamined Patent Publication No. 2008-217594

Patent Document 3: Japanese Unexamined Patent Publication No. 2008-081435

Patent Document 4: International Publication No. WO 2007/139037

Patent Document 5: International Publication No. WO 2008/053924

NON-PATENT DOCUMENT

Non-Patent Document 1: Hartenfeller, M., Schneider G. et al., "Concept of combinatorial de novo design of drug-like molecules by particle swarm optimization," Chemicalbiology & drug design 72, 16-26 (2008).

SUMMARY OF THE INVENTION

TECHNICAL PROBLEM

The conventional interaction prediction by docking is interaction prediction of known compounds, and cannot design a novel compound. For the prediction, the spatial configuration information of a protein is required. Obtaining the spatial configuration information of the protein requires considerably high cost and takes considerably long time. The conventional interaction prediction also involves problems where calculation takes considerably long time and the accuracy of the prediction is low.
In the conventional methods in Non-Patent Document 1 and other documents, de novo design is performed based on the structural similarity of ligands. However, prediction results by calculation are not experimentally verified by actually synthesizing the designed compound and performing an assay. Therefore, a problem arises in accuracy reliability.

SOLUTION TO THE PROBLEM

The inventors found that the problems described above can be solved by the pattern recognition technology to predict interaction described in Patent Document 4 and developed by themselves.

That is, the compound designing device of the present invention includes an input unit configured to receive, at least about one or more query proteins, one or more pieces of protein information corresponding to the one or more query proteins; and a processing unit configured to perform steps of:

(a) generating one or more pieces of compound information,
(b) computing a score indicating interaction potential between a compound corresponding to the compound information and each of the one or more query proteins,
(c) updating the compound information by an optimization method with reference to the score computed at step (b) such that the interaction potential increases, and
(d) repeating steps (b) and (c) a plurality of times, wherein

the score computed at step (b) is at least a score obtained by machine learning using, as teacher data, a first combination of protein information and compound information respectively corresponding to a protein and a compound causing first interaction therebetween.

In another aspect of the compound designing device of the present invention, the machine learning is a support vector machine, in which in addition to the first combination, a second combination of protein information and compound information corresponding to a protein and a compound causing second interaction therebetween is used as teacher data, a separating plane separating the first combination from the second combination is obtained, and the score indicates a distance of a combination of compound information for which the score is to be computed and protein information for which the score is to be computed from the separating plane.

In another aspect of the compound designing device of the present invention, one or more selected from the group consisting of swarm intelligence optimization, evolutionary computation, and particle swarm optimization are used as an optimization method.

Moreover, a processing unit of another compound designing device of the present invention performs, after step (c), step of

(c1) selecting a piece of compound information corresponding to the compound from pieces of compound information approximating the compound information updated at step (c) and determining the selected piece of compound information to be the updated compound information.

Another compound designing device of the present invention further incudes a memory unit, wherein the memory unit stores the updated compound information as a history, and the processing unit performs, after the step (c1), steps of:

(c2) referring to the history stored in the memory unit, and determining whether or not the selected piece of compound information is identical with the compound information in the history, and
(c3) if the selected piece of compound information is determined to be identical with the compound information in the history at step (c2), selecting another compound information and performs step (c2) again, and if selected piece of compound information is not identical with the compound information in the history at step (c2), determining the selected piece of compound information to be the updated compound information.

In another aspect of the compound designing device of the present invention, the compound information includes pieces of fragment information corresponding to fragments generated by cleaving the chemical structure of a compound based on a predetermined rule. The predetermined rule is preferably such that when a plurality of cleavage positions exist in the chemical structure of an identical compound, fragments are preferably generated based on all possible combinations of the cleavage positions.

Moreover, in another aspect of the compound designing method of the present invention, the compound information is expressed as a direct sum of vectors existing in a space in which one or more principal components resulting from a principal component analysis of the pieces of fragment information are assigned to an axis.

In another aspect of the compound designing device of the present invention, the optimization method is particle swarm optimization, the number of constitutional units of fragments of a compound to be designed is set, and the position X of a particle representing the compound information is given as follows:

[Expression 1]

$$X = \begin{pmatrix} x_{11} & \cdots & x_{1n} \\ \vdots & \ddots & \vdots \\ x_{m1} & \cdots & x_{mn} \end{pmatrix}$$

where m is the maximum number of elements of the fragments, and n is the number of constitutional units.

The velocity V of the particle is given by the following expression:

[Expression 2]

$$V = \begin{pmatrix} v_{11} & \cdots & v_{1n} \\ \vdots & \ddots & \vdots \\ v_{m1} & \cdots & v_{mn} \end{pmatrix}$$

where m is the maximum number of elements of the fragments, and n is the number of constitutional units.

In a compound designing device of another aspect of the present invention, the score computed at step (b) is obtained by combining a score obtained by machine learning using, as teacher data, the first combination of the protein information and the compound information respectively corresponding to the protein and the compound causing the first interaction therebetween with one or more selected from the group consisting of a score obtained by activity value prediction, a score obtained by selectivity prediction, a score obtained by a docking calculation, a score obtained by synthesis possibility prediction, a score obtained by ADME-Tox prediction, a score obtained by physical property prediction, and a score obtained by prediction of binding free energy obtained by molecular dynamics method. Taking the chemical characteristics of a compound into consideration makes it possible to design compounds, such as a compound having a high activity value, a compound having high selectivity to a receptor, a compound which is bonded to an active pocket of a target protein at a high probability, a compound having a high probability of organic synthesis, a compound considering pharmacokinetics/toxicity, and a compound which is energy-stable with respect to a protein which dynamically changes its structure, depending on intended use.

A compound designing method using a computer of the present invention includes steps of:

(A) inputting, at least about one or more query proteins, one or more pieces of query protein information corresponding to the one or more query proteins to an input unit of the computer;
(B) generating one or more pieces of compound information in a processing unit of the computer;
(C) computing a score indicating interaction potential between a compound corresponding to the compound information and each of the one or more query proteins in the processing unit of the computer;
(D) updating, in the processing unit of the computer, the compound information by an optimization method with reference to the score computed at score computing step (C) such that the interaction potential increases, wherein step (C) and step (D) are repeated a plurality of times, and further, the score computed at step (C) is obtained by at least machine learning using, as teacher data, a first combination of protein information and compound information respectively corresponding to a protein and a compound causing first interaction therebetween.

In another aspect of the compound designing method of the present invention, the machine learning is the support vector machine, in which in addition to the first combination, a second combination of protein information and compound information corresponding to a protein and a compound causing second interaction therebetween is used as teacher data, a separating plane separating the first combination from the second combination is obtained, and the score indicates a distance of a combination of compound information for which the score is to be computed and protein information for which the score is to be computed from the separating plane.

A computer program of the present invention which causes a computer to design a compound allows the computer to perform steps of:

(i) receiving, about one or more query proteins, one or more pieces of query protein information corresponding to the one or more query proteins;
(ii) generating one or more pieces of compound information;
(iii) computing a score indicating interaction potential between a compound corresponding to the compound information and each of the one or more query proteins;
(iv) updating the compound information by an optimization method with reference to the score computed at step (iii) so that the interaction potential increases;
(v) repeating step (iii) and step (iv) a plurality of times, wherein
the score computed at step (iii) is obtained by at least machine learning using, as teacher data, a first combination of protein information and compound information respectively corresponding to a protein and a compound causing first interaction therebetween.

In another embodiment of the compound designing program of the present invention, the machine learning is the support vector machine, in which in addition to the first combination, a second combination of protein information and compound information corresponding to a protein and a compound causing second interaction therebetween is used as teacher data, a separating plane separating the first combination from the second combination is obtained, and the score indicates a distance of a combination of compound information for which the score is to be computed and protein information for which the score is to be computed from the separating plane.

ADVANTAGES OF THE INVENTION

According to the present invention, the structure of a novel compound can be obtained based on information (e.g., a protein name, an amino acid sequence, etc.), structural information of a fragmented compound (i.e., fragments), etc. which can be easily acquired without requiring the spatial configuration information of a protein. Moreover, verification tests described in examples revealed that short-time calculation is possible, and that the resultant compound interacts with a target protein with high probability.

BRIEF DESCRIPTION OF THE DRAWINGS

[FIG. 1] FIG. 1 is a view illustrating a configuration of a frame, a unit, and a fragment of the present invention.
[FIG. 2] FIG. 2 is a view illustrating a compound designing device and a flow chart of compound design of the present invention.
[FIG. 3] FIG. 3 is view illustrating a compound designing device and a flow chart of compound design of the present invention.
[FIG. 4] FIG. 4 is a view illustrating a compound designing device and a flow chart of compound design of the present invention.
[FIG. 5] FIG. 5 is a view illustrating results of the compound design of the present invention.
[FIG. 6] FIG. 6 is a view illustrating results of the compound design of the present invention.
[FIG. 7] FIG. 7 is a plot illustrating estimated values and measured values of activity obtained by a QSAR model of a third example.
[FIG. 8] FIG. 8 is a view illustrating results of compound design of the present invention.

DESCRIPTION OF EMBODIMENTS

The present invention provides a compound designing device, a compound designing method using a computer, and a computer program configured to allow a computer to design a compound. A computer executes the computer program of the present invention, so that the computer serves as a compound designing device and can design a compound by the compound designing method of the present invention.

The compound designing device of the present invention will be described in detail below with reference to description of embodiments. The compound designing device of the present invention includes at least an input unit and a processing unit. The compound designing device of the present invention may further include a memory unit, an output unit, etc.

1. Input Unit

The input unit receives, about one or more query proteins, one or more pieces of query protein information corresponding to the one or more query proteins, and the compound designing device of the present invention receives the one or more pieces of query protein information.

That is, a target protein is regarded as a query protein, and protein information corresponding to the query protein is input to the input unit, thereby designing a compound which interacts with the query protein. The compound is designed by updating compound information in a memory unit by an optimization method. The compound information is updated by the optimization method to obtain compound information, and a compound corresponding to the obtained compound information is assumed to be a compound having high interaction potential with the query protein.

Protein information refers to information representing characteristics of a protein. Specific examples of the protein information include the name, the amino acid sequence, and the spatial configuration of a protein. The protein information is represented as a protein descriptor. Moreover, the protein information is vectorized as a multidimensional feature vector. The relative difference between two or more proteins is represented as a similarity indicator such as a distance between the vectors. An amino acid sequence is preferably used as the protein information. For example, according to a known spectrum method, an amino acid sequence is broken down into amino acid sequences of a fixed length k, and for example, the frequency of an amino acid sequence pattern of the length k can be used as a descriptor, where up to m mismatches are accepted.

The protein information input to the input unit is preferably simple information. In this case, the processing unit can perform conversion to a protein descriptor as one of steps. Specifically, a protein name or an amino acid sequence corresponding to a query protein is input to the input unit, and the processing unit generates a protein descriptor from the protein name or the amino acid sequence corresponding to the query protein.

Alternatively, the input unit may receive compound information about one or more compounds, and the compound designing device of the present invention may receive the pieces of compound information. This is because when the scaffold structure of a compound predicted to interact with a query protein is known in advance, information about the scaffold structure is input as query compound information, so that the accuracy of prediction can be increased. When a materially novel scaffold structure is known in advance, information about the materially novel scaffold structure is input as query compound information, so that a novel compound can be designed. The compound information will be described later.

2. Processing Unit

The processing unit performs steps of (a) generating one or more pieces of compound information, (b) computing a score indicating interaction potential between a compound corresponding to the compound information and each of the one or more query proteins, (c) updating the compound information by an optimization method with reference to the score computed at step (b) such that the interaction potential is increased, and (d) repeating step (b) and step (c) a plurality of times.

At step (a), at least the one or more pieces of compound information are generated. At the same time, another protein information based on the protein information corresponding to the query protein input to the input unit may be generated. When compound information is input to the input unit, another compound information may be generated based on the input compound information.

Here, compound information refers to information representing the characteristics of a compound, and specifically represents the name, the structure, the physical property, etc. of the compound. The compound information is expressed as a compound descriptor or a chemical descriptor. The compound information is vectorized as a multidimensional feature vector, and the relative difference between two or more compounds is expressed as a similarity indicator such as a distance between the vectors.

Here, the compound information includes pieces of information about fragments obtained by fragmenting the chemical structure of a compound. Fragments of the compound may be obtained by subjecting the chemical structure of a known compound to a known approach such as a retrosynthetic combinatorial analysis procedure (RECAP) rule, to fragmentation based on a special rule, or to random fragmentation. The RECAP rule is an approach that sets a cleavage position based on chemical reaction on the chemical structure of a compound. Using the pieces of fragment information obtained by the approach provides the advantage that a compound which cannot be chemically synthesized is less likely to be designed.

When a plurality of cleavage positions are located on the chemical structure of an identical compound, pieces of fragment information of fragments obtained by fragmentation at all the cleavage positions are used in the known approach. However, the inventors found that the number of types of fragments to be obtained is preferably increased in the compound design of the present invention. Therefore, the present inventors found that when a plurality of cleavage positions are located on the chemical structure of an identical compound, fragments are generated based on all the possible combinations of the cleavage positions to increase the types of the fragments.

Specifically, it is preferable that an approach that sets a cleavage position based on chemical reaction on the chemical structure of a compound (e.g., RECAP rule) be combined with an approach in which when a plurality of cleavage positions are located on the chemical structure of an identical compound, fragments are generated based on all the possible combinations of the cleavage positions. When pieces of fragment information obtained by the approach that generates fragments based on all the possible combinations of the cleavage positions is used, various compounds which are easily synthesized can be designed.

Fragments of a compound are classified into a scaffold fragment (also referred to as a mother fragment in some cases) and a substituent group fragment. The scaffold fragment and the substituent group fragment can be connected to each other based on an arbitrary rule. For example, the number of connecting bonds and a connecting pattern which are chemically valid may be set for each fragment, and the fragments may be connected to each other according to the number of connecting bonds and the connecting pattern.

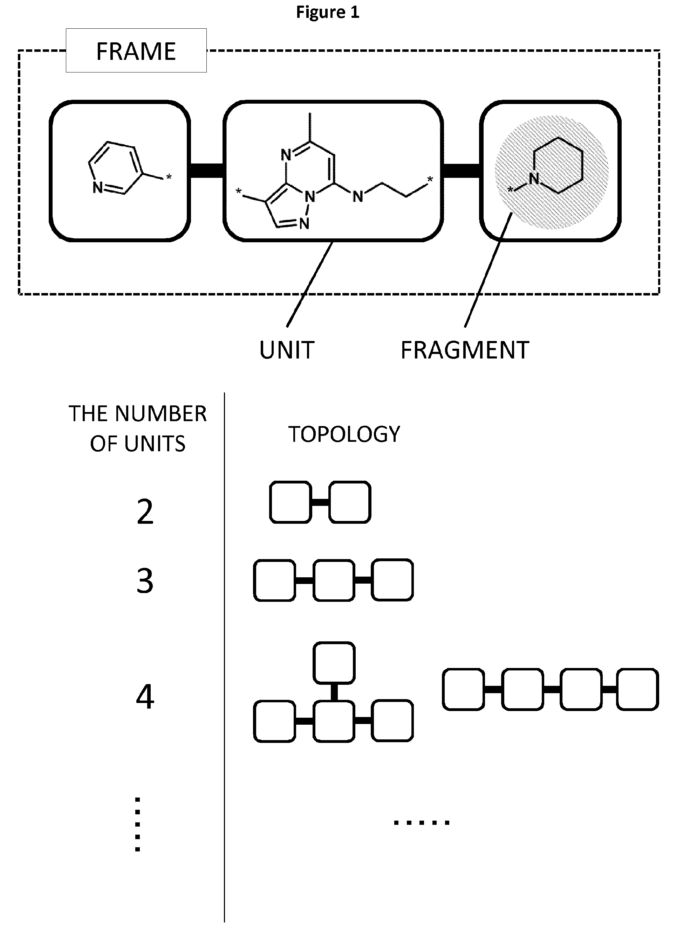

With reference to FIG. 1, a constitutional unit (i.e., a unit) and a frame which is a combination of the constitutional units will be further described in detail. Design of a compound is expressed as a frame obtained by combining fragments as constitutional units (hereinafter also referred to as units). A frame incudes one or more units, and a fragment is assigned to a unit the number of connecting bonds of which matches that of the fragment. The number of units included in a frame and the topology of the units can be freely set.

The number of units determines the topology which can be set. For example, in the case where the number of units is two, or in the case where the number of units is three, the number of topologies set for each of the cases is one. However, in the case where the number of units is four, the number of topologies which can be set is two. In the compound designing device of the present invention, the number of units of a compound to be designed is set, so that in a frame based on the topology which can be designed based on the number of units, fragments each corresponding to an associated one of the units are updated.

When the central skeleton of a compound which interacts with a query protein is known in advance, compound information is limited to that of compounds having the central skeleton, and only the substituent group fragment is updated, so that the accuracy of compound design can be increased. When a materially novel scaffold structure is known in advance, only the substituent group fragment is updated, so that a novel compound can be designed.

The compound information can be expressed by numerical representation such as continuous numerical representation, discrete numerical representation, etc. The continuous numerical representation is also referred to as continuous vector representation. In the case of the continuous numerical representation, the compound information can be expressed as a direct sum of vectors existing in a space in which one or more principal components resulting from a principal component analysis of pieces of fragment information are assigned to an axis. The discrete numerical representation is also referred to as discrete matrix representation. In the case of the discrete numerical representation, the compound information is expressed as a matrix using scores according to the frequencies of use of fragments.

In the case of the continuous vector representation, the compound information can be expressed as a direct sum of vectors existing in a space in which one or more principal components resulting from a principal component analysis of the pieces of fragment information are assigned to an axis. This is preferable because the dimension of the vector representing the compound information is low.

Specifically, compound descriptors of fragments are calculated, and a principal component analysis is performed on a group of the calculated compound descriptors of the fragments to obtain principal components. From the obtained principal components, several principal components are extracted in the descending order of the contribution ratio, and the extracted principal components are defined as vectors x representing the fragments. The direct sum of these vectors is a vector representing the compound information. Here, the number of principal components to be extracted is preferably three to 10 in consideration of the calculation efficiency. When the principal component analysis is performed on the descriptors of the fragments to obtain principal components, and principal components having high contribution ratios are extracted from the obtained principal components, a compound information vector X is generated as follows.

[Expression 3]

$$\vec{X} = \vec{x}_1 \oplus \vec{x}_2 \oplus \vec{x}_3 \oplus \cdots \oplus \vec{x}_n$$

where n is a constitutional unit (i.e., unit) in a frame.

In the continuous vector representation, vectors representing the compound information are discretely located in a space, and thus, the continuous vector representation has a feature that the updated compound information does not correspond to an actual compound. Therefore, as discrete representation, the compound information can also be represented as a matrix directly associated to the fragments.

In this case, the position X of a particle representing the compound information is given by the following expression:

[Expression 4]

$$X = \begin{pmatrix} x_{11} & \cdots & x_{1n} \\ \vdots & \ddots & \vdots \\ x_{m1} & \cdots & x_{mn} \end{pmatrix}$$

where m is the number of fragments used for compound design, and n is the number of constitutional units.

Each of elements of a matrix X indicates the selection state of fragments, where 0 represents an unselected state, and 1 represents a selected state. Since one fragment is selected for each constitutional unit, the column vector of the matrix X is a unit vector, and is thus given by the following expression:

[Expression 5]

$$x_{ij} \in (0,1),$$

$$\sum_{i=1}^{m} x_{ij} = 1, \qquad j = \{1,2,\ldots n\}$$

The protein information corresponding to the query protein input to the input unit may be converted into protein information of another format. Specifically, a protein name corresponding to a query protein is input to the input unit, and the processing unit generates amino acid sequence information from the protein name corresponding to the query protein. Based on the amino acid sequence information, the processing unit further generates a protein descriptor.

The processing unit further performs step of (b) computing a score indicating interaction potential between a compound corresponding to the compound information and each of the one or more query proteins.

Here, the score indicating interaction potential computed at step (b) is obtained by at least machine learning using, as teacher data, a first combination of protein information and compound information respectively corresponding to a protein and a compound causing first interaction therebetween. For example, support vector regression (SVR), partial least squares (PLS) regression, etc. can be used as the machine learning using the first combination as teacher data.

Further, the score indicating interaction potential computed at step (b) may be obtained by machine learning using, as teacher data, a second combination of protein information and compound information respectively corresponding to a protein and a compound causing second interaction therebetween in addition to the first combination. For example, a support vector machine (SVM), etc. can be used as the machine learning using the first combination and the second combination as teacher data.

Here, a pair (a first pair) of the protein and the compound causing the first interaction therebetween refers to, for example, a pair of a protein and a compound which are known to cause interaction. A pair (a second pair) of the protein and the compound causing the second interaction therebetween refers to, for example, a pair of a protein and a compound which are not known to cause interaction therebetween, or a random pair of a protein and a compound. The second pair is preferably a pair of a protein and a compound which are not known to cause interaction therebetween. However, it is very difficult to find such a pair in documents. Therefore, a random pair of a protein and a compound is preferably used as the second pair.

In this case, the first pair is positive example teacher data, and the second pair is negative example teacher data. The first pair and the second pair may use information obtained from documents such as theses and databases or information obtained by experimental verification.

The first pair or the first pair and the second pair are analyzed by a machine learning method such as the support vector machine, thereby constructing a learning model. Whether a pair of a query compound and a query protein belongs to the first pair or the second pair can be determined by using the learning model. Moreover, the possibility that the pair of the query compound and the query protein belongs to the first pair and the possibility that the pair of the query compound and the query protein belongs to the second pair can be expressed as scores.

The support vector machine is a type of machine learning. A space constructed by feature vectors is referred to as a feature space. By using a kernel function, the support vector machine maps vectors in a finite dimension or infinite dimension feature space, and performs linear separation on the feature space, thereby constructing a learning model. That is, a separating plane separating a plurality of vectors with a maximum margin is obtained, and the vectors are separated by the separating plane into two classes. Therefore, it is possible to determine to which class a queried vector belongs by using the separating plane.

Processes of the support vector machine will be specifically described below. First, a protein descriptor including protein information corresponding to a protein of the first pair is combined with a compound descriptor including compound information corresponding to a compound of the first pair. This combination is referred to as a first combination. Similarly, a protein descriptor including protein information corresponding to a protein of the second pair is combined with a compound descriptor including compound information corresponding to a compound of the second pair. This combination is referred to as a second combination. The kernel of the first combination and the kernel of the second combination are calculated by using these combinations, and the support vector machine is performed, so that a hyperplane classifying the first combination and the second combination is obtained.

Machine learning of the interaction relationship between a compound and a protein requires a mathematical framework in which the data representation of the compound and the data representation of the protein which are different types are integrated, and the interaction relationship of the compound and the protein is quantified. That is, when the feature vector of a compound x is X, and the feature vector of a protein y is Y, attention is focused on how the feature vector $Z(x, y)$ of a pair $(x, y)$ is constructed from the feature vector X and the feature vector Y. Here, a kernel method can be used as an effective method that integrates a compound vector and a protein vector with each other. Specifically, kernel $K_c$ of a compound and kernel $K_p$ of a protein are defined by using a compound vector $X(x)$ and a protein vector $Y(y)$ as follows:

[Expression 6]

$$K_c(x, x') = X(x)^T X(x')$$
$$K_p(y, y') = Y(y)^T Y(y')$$

Moreover, in the present invention, a synthesis method using a tensor product kernel whose effectiveness is particularly known may be used to quantify the interaction relationship between the compound and the protein. Specifically, a feature vector obtained by combining a protein descriptor and a compound descriptor is defined by the following expression:

[Expression 7]

$$Z(x,y) = X(x) \otimes Y(y)$$

where $\otimes$ is a tensor product.

When the feature vector of the pair of the compound and the protein is defined as described above, the kernel of a combination of the protein descriptor and the compound descriptor can be defined as follows.

[Expression 8]

$$K_{c-p}\big((x,y),(x',y')\big) = Z(x,y)^T Z(x',y')$$
$$= K_c(x,x') \times K_p(y,y')$$

The distance of the queried vector from the separating plane can be an indicator of likelihood of the first interaction. That is, even when vectors are classified into a class, vectors at a short distance to the separating plane are highly likely to be erroneously classified into the class, whereas vectors at a long distance to the separating plane are less likely to be erroneously classified into the class. That is, the possibility that a combination of a protein and a compound corresponding to a queried vector causes the first interaction is expressed by the distance of the vector from the separating plane. That is, at step (b), the distance of a query vector from the separating plane is computed as the score indicating interaction potential.

A computing method of the score indicating interaction potential will be specifically described below. The score indicating interaction potential is a value $s_c$ obtained by converting a decision function value x of the support vector machine with a sigmoid function. These values are defined as follows.

[Expression 9]

$$x \in [-\infty, +\infty]$$
$$s_c(x) \in [0,1]$$
$$s_c(x) = \frac{1}{1 + e^{\alpha x + \beta}}$$

Here, $\alpha$ and $\beta$ are parameters determined based on a score distribution obtained from cross-validation by the support vector machine. The parameters $\alpha$ and $\beta$ are determined such that the following function F ($\alpha$, $\beta$) is minimized, where i is the index of learning data, and $y_i$ represents whether or not the interaction occurs. If the interaction occurs, that is, if the first combination is predicted, $y_i$ is plus 1. On the other hand, if the interaction does not occur, that is, the second combination is predicted, $y_i$ is minus 1.

[Expression 10]

$$F(\alpha, \beta) = -\sum_i t_i \log(p_i) + (1 - t_i) \log(1 - p_i),$$

$$t_i = \frac{y_i + 1}{2}, \qquad p_i = \frac{1}{1 + e^{\alpha x_i + \beta}}$$

In another aspect of the present invention, the score computed at step (b) is obtained by combining at least a score obtained by machine learning using, as teacher data, the first combination of the protein information and the compound information respectively corresponding to the protein and the compound causing the first interaction therebetween with one or more selected from the group consisting of a score obtained by activity value prediction, a score obtained by selectivity prediction, a score obtained by a docking calculation, a score obtained by synthesis possibility prediction, a score obtained by ADME-Tox prediction, a score obtained by physical property prediction, and a score obtained by prediction of binding free energy obtained by molecular dynamics method. When the chemical characteristics of compounds are taken into consideration, it is possible to design compounds depending on intended use, and for example, a compound having a high activity value, a compound having high selectivity to a receptor, a compound which is bonded to an active pocket of a target protein at a high probability, a compound having a high probability of organic synthesis, a compound considering pharmacokinetics/toxicity, and a compound which is energy-stable with respect to a protein which dynamically changes its structure can be designed.

A method of combining the score obtained by machine learning using, as teacher data, the first combination of the protein information and the compound information respectively corresponding to the protein and the compound causing the first interaction therebetween with other scores is not limited. Preferably, the combination is performed by multiplication or addition of the score obtained by the machine learning by or to one or more selected from the group consisting of a score obtained by activity value prediction, a score obtained by selectivity prediction, a score obtained by a docking calculation, a score obtained by synthesis possibility prediction, a score obtained by ADME-Tox prediction, a score obtained by physical property prediction, and a score obtained by prediction of binding free energy obtained by molecular dynamics method.

In the activity value prediction, the concentration of a compound causing biological activity is predicted. If the concentration of the compound causing the biological activity is C, the score obtained by the activity value prediction is denoted by minus logC. Specific examples of the activity value prediction include a quantitative structure-activity relationship (QSAR). The QSAR is an approach that analyzes a correlation between the molecular structure characteristics of a compound and biological activity by a statistical approach such as a regression analysis to obtain a quantitative correlation equation.

A process in the case where at least a score (hereinafter referred to as "$s_c$") obtained by machine learning using, as teacher data, the first combination of the protein information and the compound information respectively corresponding to the protein and the compound causing the first interaction therebetween and a score (hereinafter referred to as "$s_q$") obtained by the activity value prediction are used as the score computed at step (b) will be described below. An evaluation function s is defined by a function of the two variables $s_c$ and $s_q$.

[Expression 11]

$$s = f(s_q, s_c)$$

Moreover, the variable $s_q$ is computed by a QSAR model, represents a value quantitatively predicting the strength of biological activity (minus logC which is a value obtained by the compound concentration C causing biological activity of interest), and can be defined as follows.

[Expression 12]

$$s_q \in [-\infty, +\infty]$$

The evaluation function s is expressed by a multiplication of $s_c$ and $s_q$ as in the following expression. For a weighting factor w, 1 or an arbitrary numeric value is set. At step (c), the compound information is updated by an optimization method to increase the evaluation function s. In the case of the following expression, the greater the numeric value of the evaluation function s, the higher the interaction potential with a query protein and the stronger the biological activity.

[Expression 13]

$$f(s_q, s_c) = w s_q s_c$$

The selectivity prediction is an approach that predicts the selectivity of bonding. When a receptor has a plurality of subtypes, there are a compound which is selectively bonded to a specific subtype and a compound which is non-selectively bonded to a plurality of subtypes. Pieces of compound information about these compounds are analyzed by machine learning such as the support vector machine, thereby constructing a learning model. Whether or not the compounds corresponding to the pieces of compound information are selectively bonded to a query protein can be predicted based on the learning model.

In the selectivity prediction using the support vector machine, a compound which is selectively bonded to a protein of a prediction target is a first compound. A compound which is non-selectively bonded to the protein of the prediction target, that is, a compound which is also non-selectively bonded to proteins other than the protein of the prediction target is a second compound. A learning model is constructed by the support vector machine in a manner similar to the above-described method, so that the possibility that the query compound belongs to the first compound or the possibility that the query compound belongs to the second compound can be expressed as a score.

A process in the case where at least a score (hereinafter referred to as "$s_c$") obtained by machine learning using, as teacher data, the first combination of the protein information and the compound information respectively corresponding to the protein and the compound causing the first interaction therebetween and a score (hereinafter referred to as "$s_s$") obtained by the selectivity prediction are used as the score computed at step (b) will be described below. An evaluation function s is defined by a function of two variables $s_c$ and $s_s$.

[Expression 14]

$$s = f(s_s, s_c)$$

The evaluation function s is expressed by a multiplication of $s_s$ and $s_c$ as in the following expression. For a weighting factor w, 1 or an arbitrary numeric value is set. At step (c), the compound information is updated by an optimization method to increase the interaction potential with a query protein. In the case of the following expression, the greater the numeric value of the evaluation function s, the higher the interaction potential with the query protein and the higher the selectivity to the query protein.

[Expression 15]

$$f(s_S, s_C) = w\sqrt{s_S s_C}$$

The term ADME-Tox is an abbreviated word of absorption, distribution, metabolism, excretion, and toxicity, and is predicted by calculating the pharmacokinetics and the toxicity in organisms as scores.

At step (c), the compound information is updated by an optimization method with reference to the score computed at step (b) so that the interaction potential with the query protein increases. An evolutionary algorithm and swarm intelligence (SI) can be used as the optimization method. Among them, particle swarm optimization (PSO) is preferably used.

The particle swarm optimization is an optimization method in which a particle swarm having a position and a velocity in a multidimensional search-space effectively and comprehensively searches a position corresponding to an optimal solution. The particles exchange information about best positions with each other. Based on the best position of each particle and the best position of the entire swarm, the position and the velocity of each particle are updated one by one. The update is repeated a plurality of times, thereby optimizing the positions of the particles.

The expression of the particle swarm optimization is generally given by the following expression:

[Expression 16]

$$\vec{X}_i(t+1) = \vec{X}_i(t) + \vec{V}_i(t+1)$$
$$\vec{V}_i(t+1) = w\vec{V}_i(t) + r_1 c_1 \{\vec{X}_i^{pbest} - \vec{X}_i(t)\} + r_2 c_2 \{\vec{X}_i^{gbest} - \vec{X}_i(t)\}$$

where $\vec{X}_i$ is the position vector of a particle i, $\vec{V}_i$ is the velocity vector of the particle i, w is an inertia parameter, $r_1$ and $r_2$ are uniform random numbers of 0-1, $c_1$ is a spring constant with respect to *pbest,* $c_2$ is a spring constant with respect to *gbest,* $\vec{X}_i^{pbest}$ is the optimal solution found by the particle i, and $\vec{X}_i^{gbest}$ is the optimal solution found by the entire swarm.

In the particle swarm optimization, the compound information is defined as the particle i. For the particle i, a score indicating interaction potential between a compound corresponding to the position of the particle i and one or more query proteins is computed at step (b). At step (c), the position of the particle i (the position vector of the particle i) and the velocity (the velocity vector of the particle i) are updated based on the score computed at step (b), an optimal score which the particle i has obtained (a best solution which the particle i has found), and an optimal score which the entire swarm has obtained (a best solution which the entire swarm has found).

Further, at step (d), step (b) and step (c) are repeated. That is, for the particle i updated at step (c), a score indicating interaction potential between a compound corresponding to the position of the particle i and the one or more query proteins is computed. Based on the computed score, the optimal score of the particle i, and the optimal score of the entire swarm, the position and the velocity of the particle i are updated. Repeating step (b) and step (c) a plurality of times updates the position and the velocity of the particle i such that the score indicating interaction potential increases, thereby eventually reaching a position corresponding to the optimal solution.

When the compound information of the present invention is expressed in continuous representation, a compound generated based on the compound information (hereinafter referred to as a vector X) updated at step (c) is a compound approximating the vector X of pieces of compound information (hereinafter referred to as vectors Y) corresponding to a compound generated by combining fragments.

Since the vectors Y which are the pieces of compound information corresponding to the compound are discontinuously scattered in a compound space, the vector X may not match the vectors Y. In this case, one of the vectors Y which approximates the vector X updated at step (c) is selected, and a score indicating the interaction potential between a compound corresponding to the compound information and each of the one or more query proteins input to the input unit is computed. The compound approximating the updated compound information is preferably a compound corresponding to one of the pieces of compound information which most approximates the updated compound information.

That is, a processing unit of another aspect of the present invention performs, after step (c), step of (c1) selecting a piece of compound information corresponding to the compound from pieces of compound information approximating the compound information updated at step (c) and determining the selected piece of compound information to be the updated compound information.

Here, at step (c1), a piece of compound information approximating the updated compound information is selected from the pieces of compound information corresponding to the compound. Preferably, a piece of compound information which most approximates the updated compound information is selected from the pieces of compound information corresponding to the compound.

The compound designing device of the present invention includes a memory unit which will be described later in addition to the input unit and the processing unit. The memory unit stores the updated compound information as a history. After step (c1), the processing unit performs step of (c2) referring to the history stored in the memory unit, and determining whether or not the selected compound information is identical with the compound information included in the history. The processing unit performs step of (c3) if the selected piece of compound information is determined to be identical with the compound information in the history at step (c2), selecting another compound information and performing step (c2) again, and if selected piece of compound information is not identical with the compound information in the history at step (c2), determining the selected piece of compound information to be the updated compound information.

At step (c1), a piece of compound information corresponding to the compound is selected from pieces of compound information approximating the updated compound information. In this case, the selected piece of compound information may be identical with a piece of compound information selected in the past. Therefore, in order to select a piece of compound information which is not identical with the piece of compound information selected in the past, the history stored in the memory unit is referred to and it is determined whether or not the piece of compound information is identical with the compound information in the memory unit. If the piece of compound information is determined to be identical with the compound information in the memory unit, another piece of compound information is selected. A piece of compound information which most approximates the updated compound information is selected from the pieces of compound information corresponding to the compound. The another compound information, which is selected in the case where the piece of compound information is determined to be identical with the compound information in the memory unit, is a piece of compound information which second most approximates the updated compound information after the piece of compound information determined to be identical with the compound information in the memory unit.

When the pieces of compound information are represented by vectors, an approximate piece of compound information is a piece of compound information whose position is close to the updated position vector. In order to compute the approximate piece of compound information, a similarity indicator such as the distance is used. The Euclidean distance, the Mahalanobis distance, the Tanimoto index, etc. are used as the similarity indicator, and preferably, the Euclidean distance can be used.

When the compound information is represented as a matrix X in the discrete representation, the general expression of the above-described particle optimization cannot be used. The velocity V of the particle X is given by the following expression:

[Expression 17]

$$V = \begin{pmatrix} v_{11} & \cdots & v_{1n} \\ \vdots & \ddots & \vdots \\ v_{m1} & \cdots & v_{mn} \end{pmatrix}$$

where m is the number of fragments used for compound design, and n is the number of constitutional units.

Steps of updating the compound information in the case where the compound information is expressed as the matrix X will be specifically described. First, in generating the compound information, fragments are randomly selected so that each particle is located at a position X0. The initial speed V0 is also randomly set.

Next, a predicted score of interaction between a compound to which the position of the particle corresponds and a query protein is calculated. Based on the results of calculation of the score, $V_{pbest}$ and $V_{gbest}$ are obtained. Here, $V_{pbest}$ is defined by a velocity matrix corresponding to the best solution found by each particle, and $V_{gbest}$ is defined by a velocity matrix corresponding to the best solution in the entire swarm.

Based on the following expression, $V_{t+1}$ is updated.

[Expression 18]

$$V_{t+1} = w \times V_t + r_1 c_1 \times V_{pbest} + r_2 c_2 \times V_{gbest}$$

where w is the inertia constant, $r_1$ and $r_2$ are uniform random numbers of 0-1, $c_1$ and $c_2$ are constants representing the magnitude at which a particle moves toward the best solution.

Moreover, $X_{t+1}$ is probabilistically updated by roulette selection, ranking selection, tournament selection, elitist selection, etc. The roulette selection is preferably used as a selection method. The selection probability $Pr(X_t)$ of the position $X_t$ by the Boltzmann distribution is given by the following expression:

[Expression 19]

$$Pr(X_t) = \frac{\exp(V_t/T)}{\sum \exp(V_t/T)}$$

Here, T (>0) is a constant determining the degree of probability which is referred to as a temperature parameter. At limit T→0, T is updated with X so that V is maximum.

[Expression 20]

$$X_{t+1} = arg \max_{X_t} V_t$$

When the particle swarm optimization is used, a compound corresponding to the position of the updated particle i may be a compound identical with a compound selected in the past. Thus, the history is referred to determine whether or not the compound corresponding to the position of the updated particle i is identical with the compound selected in the past. If the compound corresponding to the position of the updated particle i is determined to be identical with the compound selected in the past, a position approximating the position of the updated particle i and corresponding to another compound is determined to be the position of a new particle i.

Moreover, when the particle swarm optimization is used as the optimization method of the present invention, particles may converge at a local solution at an early stage. In this case, a problem arises where a global optimal solution may be missed. Thus, the behavior of the particles is adjusted so that they do not converge at the local solution, and a further measure allowing the particles to escape from convergent state is preferably taken.

Therefore, the particle used in the particle swarm optimization is divided. Specifically, a particle swarm is divided into a plurality of groups, one of which includes only particles close to one another. Information exchange between the particles is performed only in the same group, and good solutions of best solutions *gbest* between adjacent ones of the groups are rewritten.

Moreover, in order to prevent localization of the groups, a first hierarchy in which information exchange is performed in the same group is set, a second hierarchy in which a global search is performed is set, and setting is made such that when the solution of the second hierarchy is good, the solution of the first hierarchy is rewritten. The global diversity of the best solutions *gbest* of the second hierarchy is maintained through initialization performed at an arbitrary cycle. By applying such division hierarchical particle swarm optimization to the present invention, the diversity of the particles is maintained, and the compound information is updated to more suitable compound information.

The number of repetitions of step (b) and step (c) at step (d) can be predetermined. That is, step (e) can be a step of executing step (b) and step (c) when the number of times of performing step (b) and step (c) is less than the default number of times, and terminating the process when the number of times of performing step (b) and step (c) reaches the default number of times.

Moreover, a default value of the score indicating the interaction potential is predetermined, and step (b) and step (c) may be repeated until the score reaches the default value. That is, step (e) can be a step of performing step (b) and step (c) when the score representing the interaction potential is less than the default value, and terminating the process when the score indicating the interaction potential reaches the default value.

Step (e) may be performed before step (b) or may be performed before step (c).

Moreover, in another example of the present invention, when step (c1), step (c2), or step (c3) is performed after step (c), step (c1), (c2), or (c3) is repeated after step (c). That is, step (d) can be a step of repeating step (b), step (c), and step (c1) a plurality of times, a step of repeating step (b), step (c), step (c1), and step (c2) a plurality of times, or a step of repeating step (b), step (c), step (c1), step (c2), and step (c3) a plurality of times.

### 3. Memory Unit

The compound designing device of the present invention may include a memory unit. The memory unit stores learning model obtained by at least machine learning using, as teacher data, a first combination of protein information and compound information respectively corresponding to a protein and a compound causing first interaction therebetween. At step (b), the processing unit accesses the memory unit storing the learning model, and computes a score.

Moreover, in addition to the learning model, the memory unit may store prediction models of the chemical characteristics of compounds such as an activity value prediction model, a selectivity prediction model, a docking calculation model, a synthesis possibility prediction model, an ADME-Tox prediction model, a score obtained by physical property prediction, a molecular dynamics method model, etc. At step (b*), the processing unit accesses the memory unit storing the prediction models, and computes a score.

In another example of the present invention, the memory unit stores compound information selected at step (b1) as a history. Moreover, at step (d1), the history stored in the memory unit is referred to, and it is determined whether or not the selected compound information is identical with the compound information in the history. If the selected compound information is determined to be identical with the compound information in the history at step (d2), another compound information is selected at step (d3) and step (d2) is performed again. If the selected compound information is determined not to be identical with the compound information in the history at step (d2), the selected compound information is determined to be the updated compound information. Further, at step (d4), the compound information selected at step (d3) is stored in the memory unit as a history.

The memory unit may store a database including pieces of fragment information included in the compound information. At step (a), the processing unit can access the fragment database stored in the memory unit, and can generate one or more pieces of compound information from the pieces of fragment information included in the library.

### 4. Output Unit

The compound designing device of the present invention may include an output unit. The output unit outputs compound information determined by the processing unit to most probably interact with a query protein or a chemical structure corresponding to the compound information. Here, a compound output from the output unit is not limited to a compound having a known chemical structure, and the output unit may also output a compound having a novel chemical structure.

The present invention also provides a compound designing method using a computer and a computer program allowing the computer to design a compound.

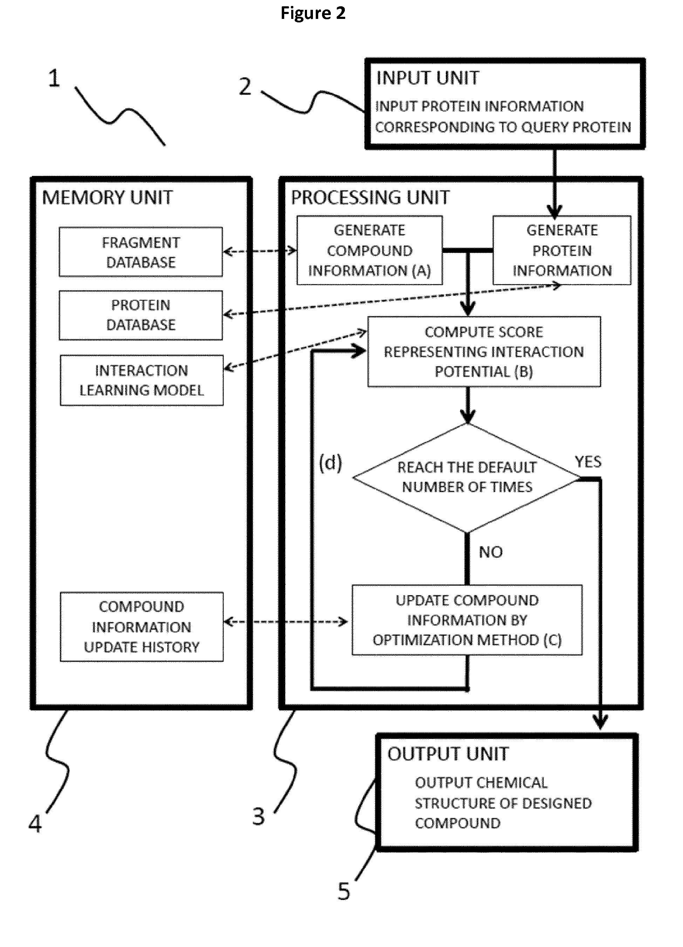

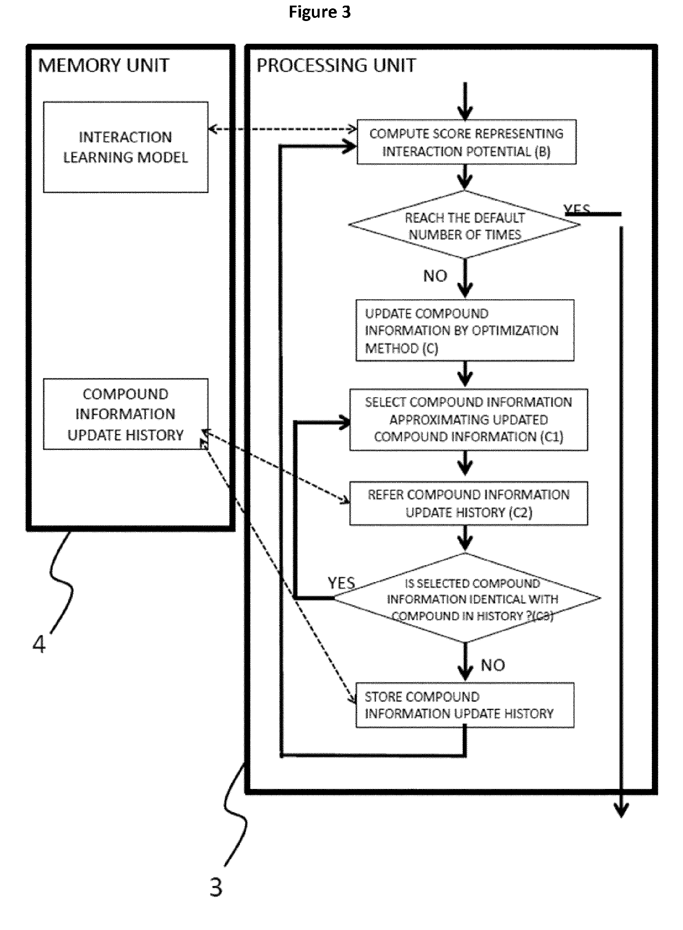

With reference to the drawings, the present invention will be further described in detail. FIG. 2 shows an embodiment and a flow chart of a compound designing device 1 of the present invention. The compound designing device 1 includes an input unit 2, a processing unit 3, a memory unit 4, and an output unit 5. FIG. 3 is a view illustrating the flow chart of processes performed by the processing unit 3 of the compound designing device 1 of the present invention and the relationship between each of the process and the memory unit 4.

Protein information corresponding to a query protein is input to the input unit 2 of the compound designing device 1. Based on the input protein information, the processing unit 3 further generates another protein information corresponding to the query protein. Here, the protein name of the query protein is input to the input unit 2. The processing unit 3 refers to a protein database stored in the memory unit 4 to search for an amino acid sequence corresponding to the protein name. For example, according to a known spectrum method, the amino acid sequence is broken down to amino acid sequences of a fixed length k. The frequency of an amino acid sequence pattern of the length k is generated as a protein descriptor, where up to m mismatches are accepted

The memory unit 4 stores a fragment database. Fragment descriptors obtained by fragmenting the chemical structures of known compounds at cleavage positions according to the RECAP rule and the chemical structures are recorded in the fragment database. For a chemical structure having a plurality of cleavage positions, fragment descriptors and chemical structures obtained from all possible combinations of the cleavage positions are recorded.

Based on a default frame, the processing unit 3 randomly generates a compound descriptor from the fragment descriptors and the chemical structures recorded in the fragment database.

The processing unit 3 computes a score indicating interaction potential between a compound corresponding to compound information and the query protein. The memory unit 4 stores an interaction learning model. The interaction learning model is obtained by at least machine learning using, as teacher data, a combination of a protein and a compound causing first interaction therebetween, that is, a protein and a compound which are known to cause interaction therebetween as a positive example.

When the support vector machine is used as the machine learning, a combination of a protein and a compound causing second interaction therebetween is also used as the teacher data in addition to the protein and the compound causing the first interaction therebetween. In this case, the interaction learning model is a model in which a feature vector obtained by combining descriptors of a protein and a compound which are known to cause interaction therebetween as a positive example and a feature vector obtained by randomly combining descriptors of a protein and a compound as a negative example are used, and a separating plane separating the positive example and the negative example from each other is constructed in a feature space by the support vector machine, wherein based on the distance from the separating plane, the score indicating the interaction potential is computed.

The processing unit 3 refers to the interaction learning model stored in the memory unit 4, calculates a descriptor of a compound corresponding to generated plurality of pieces of compound information, and computes the score indicating the interaction potential based on the distance of the feature vector to the separating plane, where the feature vector is obtained by combining the descriptors of the compound with the descriptor of the query protein. When the feature vector is classified into the positive example side, it is predicted that the interaction potential increases as the distance from the separating plane increases. In contrast, when the feature vector is classified into the negative example side, it is predicted that the interaction potential increases as the distance from the separating plane decreases in the negative example.

Subsequently, the processing unit 3 verifies whether or not the number of times of updating the compound information has reached a default number of times. If the number of times of updating the compound information is less than the default number of times, the compound information is updated by an optimization method based on the score indicating the interaction potential.

If the number of times of updating the compound information has reached the default number of times, the output unit 5 outputs a chemical structure of a compound corresponding to the updated compound information.

In the processing unit 3, if the number of times of updating the compound information is less than the default number of times, the compound information is updated by an optimization method. The particle swarm optimization is used as the optimization. For each piece of compound information, the position and the velocity are updated by the particle swarm optimization based on the score indicating its interaction potential.

The updated compound information is recorded in a compound information update history of the memory unit 4, and is processed such that a compound identical with a compound selected in the past is not selected.

Subsequently, with reference to FIG. 3, a flow following the verification that the number of times of updating the compound information has reached the default number of times in an embodiment will be described in detail, wherein in the embodiment, the compound information is represented as a direct sum of vectors existing in a space in which one or more principal components resulting from a principal component analysis of pieces of fragment information are assigned to an axis.

If the number of times of updating the compound information has not reached the default number of times, the processing unit 3 updates the compound information by an optimization method. In the present embodiment, the space in which compound information exists is continuous, whereas pieces of compound information having a corresponding compound exist discretely in a space. Therefore, the compound may not correspond to the updated compound information. Thus, the processing unit 3 selects a piece of compound information corresponding to the compound from pieces of compound information approximating the updated compound information.

Moreover, the processing unit 3 refers to the update history of the compound information stored in the memory unit 4, and verifies whether or not the compound information is identical with the compound information in the update history. If the compound information is identical with the compound information in the update history, the process returns to the step of selecting compound information to select another compound information. If the compound information is not identical with the compound information in the update history, the compound information is recorded in the update history of the compound information.

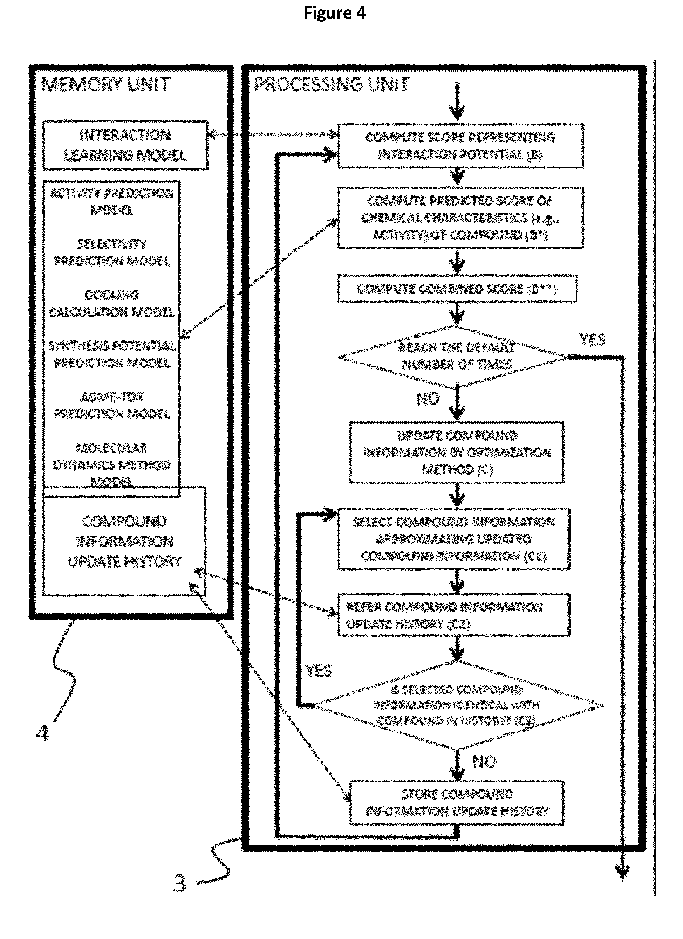

FIG. 4 shows another embodiment of the compound designing device of the present invention. The memory unit 4 stores models indicating the chemical characteristics of compounds such as an activity value prediction model, a selectivity prediction model, etc. in addition to the interaction learning model. The processing unit 3 computes (b*) a score with reference to the models indicating the chemical characteristics of a compound corresponding to compound information and stored in the memory unit 4. With reference to a score (b**) obtained by combining the score (b*) and a score indicating the interaction potential, the compound information will be updated by an optimization method in the subsequent steps.

The present invention provides a method of designing a compound by performing the above-described processes by using a computer.

That is, the compound designing method using a computer of the present invention includes steps of:

(A) inputting, at least about one or more query proteins, one or more pieces of query protein information corresponding to the one or more query proteins to an input unit of the computer;
(B) generating one or more pieces of compound information in a processing unit of the computer;
(C) computing a score indicating the interaction potential between a compound corresponding to the compound information and each of the one or more query proteins in the processing unit of the computer;
(D) updating, in the processing unit of the computer, the compound information by an optimization method with reference to the score computed at score computing step (C) such that the interaction potential increases, wherein step (C) and step (D) are repeated a plurality of times, and further, the score computed at step (C) is obtained by at least machine learning using, as teacher data, a first combination of protein information and compound information respectively corresponding to a protein and a compound causing first interaction therebetween.

In another embodiment of the compound designing method of the present invention, the machine learning is the support vector machine, in which in addition to the first combination, a second combination of protein information and compound information corresponding to a protein and a compound causing second interaction therebetween is used as teacher data, a separating plane separating the first combination from the second combination is obtained, and the score indicates a distance of a combination of compound information for which the score is to be computed and protein information for which the score is to be computed from the separating plane.

In another embodiment of the compound designing method of the present invention, one or more selected from the group consisting of swarm intelligence optimization, evolutionary computation, and the particle swarm optimization are used.

Another embodiment of the compound designing method of the present invention includes, after step (D), step of

(D1) selecting a piece of compound information corresponding to the compound from pieces of compound information approximating the updated compound information at step (D), and determining the selected compound information to be the updated compound information.

Further, in another embodiment of the compound designing method of the present invention, the memory unit of the computer stores the updated compound information as a history, and the method includes, after step (D1), steps of:

(D2) referring to the history stored in the memory unit and determining whether or not the selected compound information is identical with the compound information included in the history; and
(D3) if the selected compound information is determined to be identical with the compound information in the history at step (D2), selecting another piece of compound information and performing step (D2) again, and if the selected compound information is determined not to be identical with the compound information in the history, determining the selected compound information to be the updated compound information, wherein

steps (D2) and (D3) are performed by the processing unit of the computer.

In another embodiment of the compound designing method of the present invention, the compound information includes pieces of fragment information corresponding to fragments generated by cleaving the chemical structure of a compound based on a predetermined rule. The predetermined rule is preferably such that when a plurality of cleavage positions exist in the chemical structure of an identical compound, fragments are preferably generated based on possible combinations of the cleavage positions.

Moreover, in another embodiment of the compound designing method of the present invention, the compound information is expressed as a direct sum of vectors existing in a space in which one or more principal components resulting from a principal component analysis of the pieces of fragment information are assigned to an axis.

In another embodiment of the compound designing method of the present invention, the particle swarm optimization is used as the optimization method, the number of constitutional units of fragments of a compound to be designed is set, and the position X of a particle representing the compound information is given by the following expression:

[Expression 21]

$$X = \begin{pmatrix} x_{11} & \cdots & x_{1n} \\ \vdots & \ddots & \vdots \\ x_{m1} & \cdots & x_{mn} \end{pmatrix}$$

where m is the maximum number of elements of the fragments, and n is the number of constitutional units.

The velocity V of the particle is given by the following expression:

[Expression 22]

$$V = \begin{pmatrix} v_{11} & \cdots & v_{1n} \\ \vdots & \ddots & \vdots \\ v_{m1} & \cdots & v_{mn} \end{pmatrix}$$

where m is the maximum number of elements of the fragments, and n is the number of constitutional units.

The present invention provides a program configured to allow a computer to perform processes relating to compound design of the above-described method. The computer performs the program, so that the computer functions as a compound designing device.

That is, the computer program of the present invention which allows a computer to design a compound allows the computer to perform steps of:

(i) receiving, about one or more query proteins, one or more pieces of query protein information corresponding to the one or more query proteins;
(ii) generating one or more pieces of compound information;
(iii) computing a score indicative of interaction potential between a compound corresponding to the compound information and each of the one or more query proteins;
(iv) updating the compound information by an optimization method with reference to the score computed at step (iii) so that the interaction potential increases;
(v) repeating step (iii) and step (iv) a plurality of times, wherein
the score computed at step (iii) is obtained by at least machine learning using, as teacher data, a first combination of protein information and compound information respectively corresponding to a protein and a compound causing first interaction therebetween.

In another embodiment of the compound designing program of the present invention, the machine learning is the support vector machine, in which in addition to the first combination, a second combination of protein information and compound information corresponding to a protein and a compound causing second interaction therebetween is used as teacher data, a separating plane separating the first combination from the second combination is obtained, and the score indicates a distance of a combination of compound information for which the score is to be computed and protein information for which the score is to be computed from the separating plane.

In another embodiment of the compound designing program of the present invention, one or more selected from the group consisting of the swarm intelligence optimization, the evolutionary computation, and the particle swarm optimization are used an optimization method.

Another embodiment of the compound designing program of the present invention includes, after step (iv), step of

(iv-1) selecting a piece of compound information corresponding to the compound from pieces of compound information approximating the compound information updated at step (iv), and determining the selected compound information to be the updated compound information.

Further, in another embodiment of the compound designing program of the present invention, the memory unit of the computer stores the updated compound information as a history, and the method includes, after step (iv-1), steps of:

(iv-2) referring to the history stored in the memory unit and determining whether or not the selected compound information is identical with the compound information included in the history, and
(iv-3) if the selected compound information is determined to be identical with the compound information in the history at step (iv-2), selecting another piece of compound information and performing step (iv-2) again, and if the selected compound information is determined not to be identical with the compound information in the history, determining the selected compound information to be the updated compound information, wherein
steps (iv-2) and (iv-3) are performed by the processing unit of the computer.

In another embodiment of the compound designing program of the present invention, the compound information includes pieces of fragment information corresponding to fragments generated by cleaving the chemical structure of a compound based on a predetermined rule. The predetermined rule is preferably such that when a plurality of cleavage positions exist in the chemical structure of an identical compound, fragments are preferably generated based on possible combinations of the cleavage positions.

Moreover, in another embodiment of the compound designing program of the present invention, the compound information is expressed as a direct sum of vectors existing in a space in which one or more principal components resulting from a principal component analysis of pieces of the fragment information are assigned to an axis.

In another embodiment of the compound designing program of the present invention, the particle swarm optimization is used as the optimization method, the number of constitutional units of fragments of a compound to be designed is set, and the position X of a particle representing the compound information is given by the following expression:

[Expression 23]

$$X = \begin{pmatrix} x_{11} & \cdots & x_{1n} \\ \vdots & \ddots & \vdots \\ x_{m1} & \cdots & x_{mn} \end{pmatrix}$$

where m is the maximum number of elements of the fragments, and n is the number of constitutional units.

The velocity V of the particle is given by the following expression:

[Expression 24]

$$V = \begin{pmatrix} v_{11} & \cdots & v_{1n} \\ \vdots & \ddots & \vdots \\ v_{m1} & \cdots & v_{mn} \end{pmatrix}$$

where m is the maximum number of elements of the fragments, and n is the number of constitutional units.

EXAMPLES

With reference to examples, the present invention will be further described in detail. The present invention is not limited to the following examples.

First Example

Cross-validation was performed, where 4,700 known active compounds of cyclin-dependent kinase 2 (CDK2) were used. Six hundred of 4,700 compounds were used as learning data to configure an interaction learning model. Descriptors of the active compounds were calculated by a DRAGON6 program. Moreover, descriptors of proteins to which the active compounds were targeted were calculated by a spectrum method.
Here, descriptors relating to the structure and physical properties of the compounds were calculated by DRAGON6 ver.6.0.30 (Talete srl) as the compound descriptors. Specifically, 894 types of descriptors in total of block 1-2 (constitutional descriptors and ring descriptors), block 4-5 (walk and path counts and connectivity indices), block 8 (2D autocorrelations), block 10-11 (P_VSA-like descriptors and ETA indices), block 22-24 (atom-centered fragments, atom-type E-state indices, and CATS 2D), and block 28 (molecular properties) were calculated.
The descriptors of each pair causing interaction were combined with each other to configure a feature vector, and the interaction learning model was configured by a LIBSVM program as the support vector machine.
Six hundred of the 4,700 compounds other than the learning data were used to create pieces of fragment information. The pieces of fragment information were combined, thereby configuring compound information. A fragment generation approach (1) based on the known RECAP rule was compared with a fragment generation approach (2) in which when a plurality of cleavage positions are located on the chemical structure of an identical compound based on the RECAP rule, fragments are generated based on all the possible combinations of the cleavage positions. As a result, the following fragments and combinations of the fragments were obtained.

[Table 1]

|  | (1) | (2) |
|---|---|---|
| Fragment Having One Connecting Bond | 308 | 879 |
| Fragment Having Two Connecting Bond | 340 | 1175 |
| The Number of Combinations of Fragments | $1.92 \times 10^5$ | $1.07 \times 107$ |

A compound is expressed as a combination of fragments. Thus, it was found that the approach (2) produces 56 times as many compounds as the approach (1). This shows the possibility that when compound information is created by the approach (2), a compound can be designed at higher accuracy than when the compound information is created by the approach (1).
Subsequently, the processing unit calculated descriptors of the fragments by DRAGON6ver.6.0.30 (Talete srl) in a manner similar to the above-described method, thereby forming a fragment database.
Moreover, a frame in which three constitutional units are in serial were selected as a frame of a compound to be designed.

Ten sets of the above-described verification data and the learning data were created, and verification was repeatedly performed.

A query protein was input as a cyclin-dependent kinase 2 (CDK2). The processing unit searched for an amino acid sequence of the CDK2, and based on the amino acid sequence of the CDK2, protein descriptors of the CDK2 were calculated by a spectrum method.

The processing unit performed a principal component analysis on all the fragment descriptors, and extracted three principal components in the order of descending contribution ratio. Further, fragments corresponding to the frame were randomly selected, thereby generating vectors representing compound information. The number of the vectors representing the compound information was 990.

In the interaction learning model, the distance of each of feature vectors from the separating plane was calculated as a score, where the feature vectors were obtained by combining descriptors of chemical substances each corresponding to an associated one of the 990 pieces of compound information and the protein descriptors of the CDK2. The position and the velocity of each vector representing the compound information were updated by the particle swarm optimization. The default number of times of update was 5000.

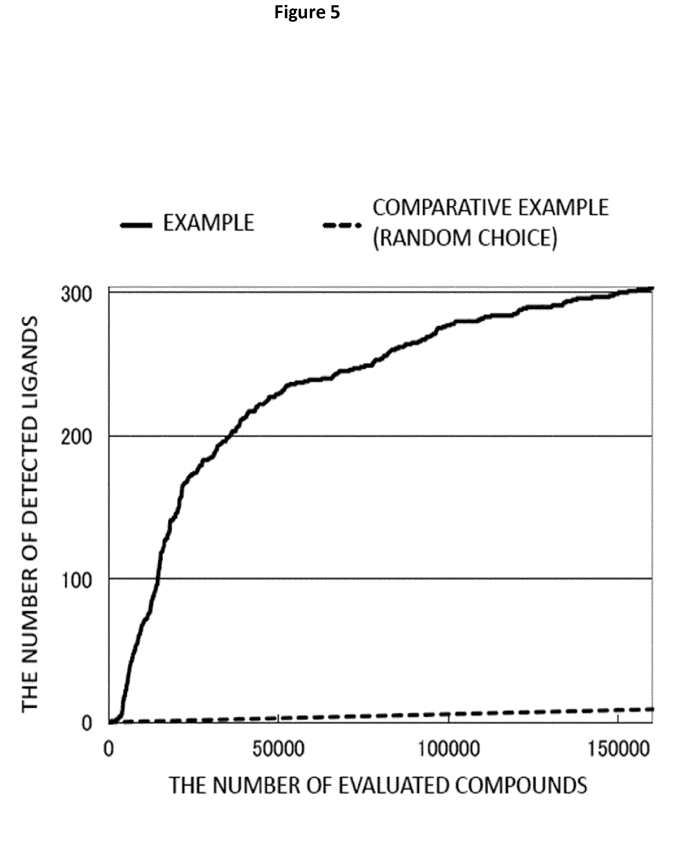

Of the output designed compounds, the number of compounds whose chemical structures are identical with the chemical structure of a compound known as a CDK2 ligand was counted. The results are shown in FIG. 5. This shows that it is possible to design compounds at remarkably high efficiency compared to a comparative example in which the update of the compound is randomly selected.

Second Example

A $\beta 2$ adrenalin receptor ($\beta 2AR$) was used as a query protein, and an antagonist of the $\beta 2$ adrenalin receptor ($\beta 2AR$) was designed according to the present invention. A frame in which three constitutional units are in series was selected as a frame of a compound to be designed. Note that a central constitutional unit was fixed as a scaffold, and only substituent fragments (R1 and R2) were updated.

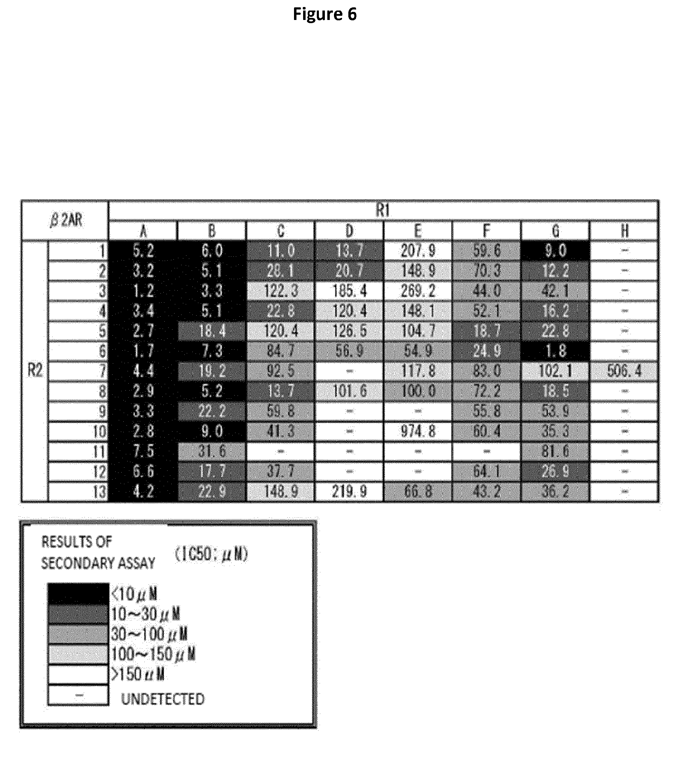

The designed compounds (R1:A-H, R2:1-13) were assayed to investigate whether or not the compounds designed according to the present invention interact with the $\beta 2AR$, which is the query protein. The results are shown in FIG. 6. When the threshold of the hit in the assayed compounds was set to less than 30 $\mu M$, a very high hit rate of 38% was obtained. When the threshold of the hit was set to less than 150 $\mu M$, a higher hit rate of 74% was obtained.

Third Example

In another aspect of the present invention, at step (b), a compound was designed with reference to a score obtained by multiplying a score obtained by machine learning using, as teacher data, a first combination of protein information and compound information respectively corresponding to a protein and a compound causing first interaction therebetween and a score obtained by activity value prediction. A compound database targeting CDK2 and V1b was used.

First, as a model of the activity value prediction, a QSAR model was constructed. For the construction of the QSAR model, a linear $\varepsilon$-support vector regression (SVR) method was used. Calculation parameters were set such that the value of the cross-validation (5-fold) was maximum. Table 2 shows the calculation conditions of CDK2 and V1b and the results.

[Table 2]

| Target | CDK2 | V1b |
|---|---|---|
| The Number of Ligands | 1886 | 350 |
| Range of Activity Value | 0.3 nM-1.52 $\mu M$ | 0.03 nM-225.6 $\mu M$ |
| Correlation Coefficient | 0.9158 | 0.9586 |
| Cross-Validation Value | 0.7816 | 0.7721 |

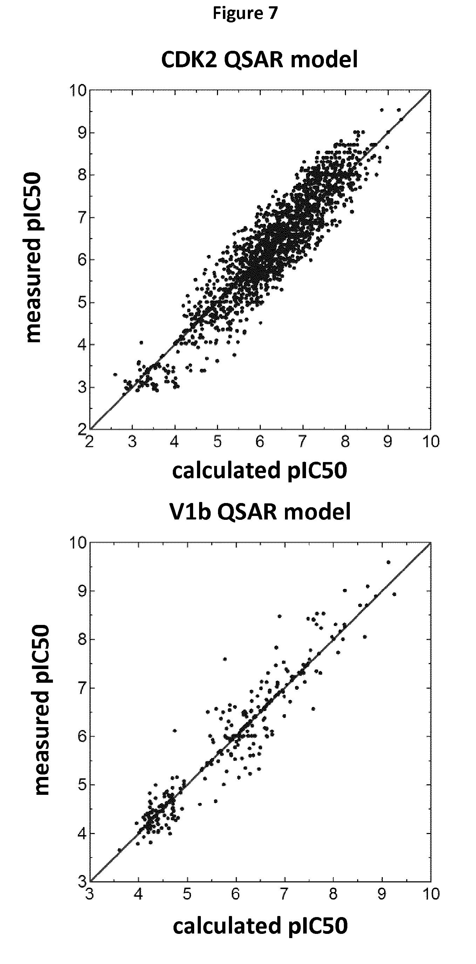

FIG. 7 is a plot of calculated values (predicted activity values) obtained by the constructed QSAR model and measured values. It is shown that the closer to the straight line the compound is plotted, the closer the estimated value and the measured value of the activity to each other.

Subsequently, a fragment database was constructed. For CDK2 and V1b, 658 compounds and 350 compounds were respectively obtained by removing compounds having low frequency of occurrence from the compounds used to construct the QSAR model. The 658 compounds and 350 compounds were fragmented in a manner similar to the first example, thereby obtaining fragments and combinations of the fragments as shown in Table 3.

[Table 3]

|  | CDK2 | V1b |
|---|---|---|
| Fragment Having One Connecting Bond | 617 | 1795 |
| Fragment Having Two Connecting Bond | 595 | 2008 |
| The Number of Combinations of Fragments | $6.46 \times 10^6$ | $1.24 \times 10^7$ |

CDK2 or V1b which is a query protein was input, the descriptors of each pair causing interaction were combined with each other to configure a feature vector, and the interaction learning model was configured by the LIBSVM program as the support vector machine.

The processing unit performed a principal component analysis on all the fragment descriptors, and extracted three principal components in the order of descending contribution ratio. Further, fragments corresponding to the frame were randomly selected, thereby generating vectors representing compound information. The number of the vectors representing the compound information was 658 for CDK2, and the number of the vectors representing the compound information was 350 for V1b.

Moreover, in the processing unit, at step (b), the evaluation function s was computed as follows. Here, $s_q$ is a predicted activity value obtained by the QSAR model, and $s_c$ is the distance of each of feature vectors from the separating plane calculated as a score, where the feature vectors are obtained by combining the descriptors of chemical substances each corresponding to an associated one of the pieces of compound information and the protein descriptors of the query protein in the interaction learning model. The weighting factor w was set to 1.

[Expression 25]

$$S = W S_q S_c$$

Moreover, the processing unit updated the position and the velocity of each vector representing the compound information by the particle swarm optimization such that the evaluation function s was maximized. The number of particles was 128 for each vector, and the default number of times of updating was 10,000.

In the example, F-measure (F value) was used as an indicator of performance evaluation. Typical evaluation indicators of prediction accuracy of binary classification problem include Recall (reproduction rate) and Precision (matching rate), and the F value has the advantage that the values of the reproduction rate and the matching rate which are in a trade-off relationship can be evaluated with one indicator value. Each of evaluation values is given by the following expression:

[Expression 26]

$$Recall = \frac{tp}{tp + fn}, \; Precision = \frac{tp}{tp + fp},$$

$$F - measure = \frac{2 \cdot Precision \cdot Recall}{Precision + Recall}$$

The reproduction rate represents a proportion of ligands determined to be correctly positive by calculation with respect to known ligands. On the other hand, the matching rate represents a proportion of the known ligands contained in a compound which is predicted to be positive by calculation. The F value is defined by a harmonic mean of the matching rate and the reproduction rate. As the values of both of the matching rate and the reproduction rate increase, the F value exhibits a higher value while approximating 1. Here, tp, fin, fp, tn represent the number of compounds corresponding to logical connections (TP, FN, FP, TN) of Table 4. Table 4 is a $2 \times 2$ contingency table illustrating the relationship between the experimental result and the calculation result of the biological activity.

[Table 4]

| | | Experimental Results | |
|---|---|---|---|
| | | Activity Found | No Activity Found |
| Prediction Results | Positive | True Positive (TP) | False Positive (FP) |
| | Negative | False Negative (FN) | True Negative (TN) |

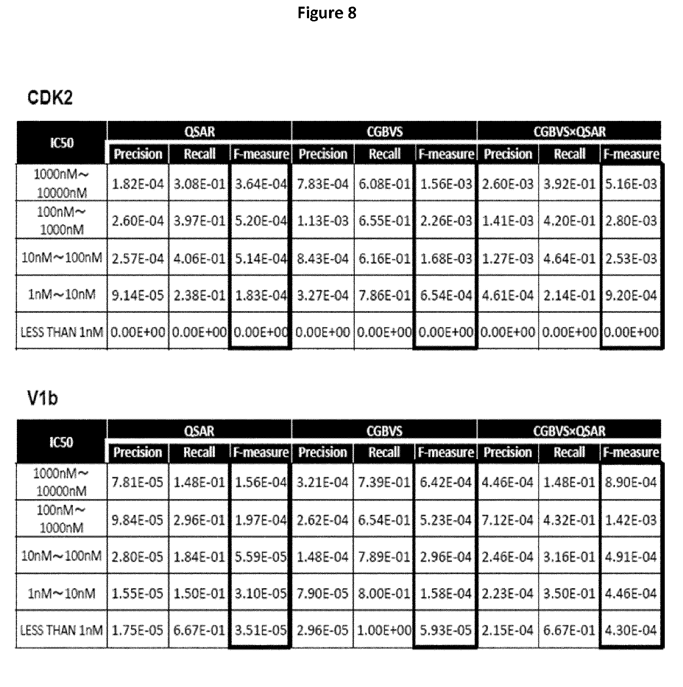

The results are shown in FIG. 8. Prediction accuracy was computed for each of ranges of activity values obtained by the experiment of the known ligands, and was summarized for each of targets in the table. An approach using the QSAR model and the interaction learning model in combination expressed higher performance of designing a compound than the other approaches. In particular, many successful cases of designing known compounds having high activity value level by experiments resulted. This suggests that the occurrence of false positive compounds is reduced, and highly active compounds can be more efficiently designed.

Fourth Example

Moreover, in order to design a novel compound including a target molecule as a β2 adrenalin receptor and having selective activity to the other adrenergic receptors, a compound was designed based on a score obtained by multiplying a score obtained at step (b) by machine learning using, as teacher data, a first combination of protein information and compound information respectively corresponding to a protein and a compound causing the first interaction therebetween by the score obtained by selectivity prediction.

First, a selectivity prediction model was constructed. The adrenergic receptors are categorized into three types, i.e., α1, α2, and β, which are further categorized into three subtypes, i.e., α1A, α1B, α1D, α2A, α2B, α2C, and β1, β2, β3, respectively. The number of known ligands of each subtype is shown in Table 5. The data is derived from commercially available compound databases, public databases (e.g., ChEMBL), and databases collected from theses and patents by the inventors. In any case, a compound exhibiting target activity with IC50 value of 30 μM or less determined by assay was defined as a known ligand.

[Table 5]

| β2AR | Subtype | The Number of Known Ligands |
|---|---|---|
| α1 | α1A | 17338 |
| | α1B | 14865 |
| | α1D | 14953 |
| α2 | α2A | 14486 |
| | α2B | 13927 |
| | α2C | 14678 |
| β | β1 | 19916 |
| | β2 | 23003 |
| | β3 | 27637 |

Of 23003 known ligands registered as β2, 3669 ligands were selective with respect to other subtypes. On the other hand, the other 19334 ligands were redundant with respect to the other subtypes of the known ligands, and were non-selective. In the example, a SVM model for selectivity prediction was constructed, where 3669 known ligands were used as positive data, and 19334 known ligands were used as negative data. The LIBSVM program was used for creation of the model and score calculation.

Subsequently, a fragment database was constructed. All of the 3669 known ligands were fragmented in a manner similar to that in the first example. The number of fragments each having one connecting bond was 8976, the number of fragments each having two connecting bonds was 9715, and the number of compounds obtained by combining these fragments was $2.7 \times 10^{10}$. The performance of each approach was evaluated based on whether or not the 3669 known ligands contained in the compounds were detectable by a small number of steps.

Moreover, in the processing unit, at step (b), the evaluation function s was computed as follows. Here, $s_s$ is a selectivity probability value obtained by the selectivity prediction model, and $s_c$ is the distance of each of feature vectors from the separating plane calculated as a score, where the feature vectors are obtained by combining the descriptors of chemical substances each corresponding to an associated one of the pieces of compound information and the protein descriptors of the query protein in the interaction learning model. The weighting factor w was set to 1.

[Expression 27]

$$S = w\sqrt{s_s s_c}$$

Moreover, the processing unit updated the position and the velocity of each vector representing the compound information by the particle swarm optimization such that the evaluation function s was maximized. The number of particles was 128 for each vector, and the default number of times of updating was 10,000.

In the example, the F-measure (F value) was used as an indicator of performance evaluation. The results of verification targeting the β2 adrenalin receptor are shown in Table 6.

[Table 6]

| First Example | | | Second Example | | |
|---|---|---|---|---|---|
| Precision | Recall | F-measure | Precision | Recall | F-measure |
| 2.67E-04 | 6.38E-02 | 5.32E-04 | 3.53E-04 | 7.88E-02 | 7.034E-04 |

The results of the approach of the fourth example and the results of the approach of the first example were compared to each other. The approach of the fourth example in which the selectivity prediction model and the interaction learning model were combined with each other exhibited higher performance of designing compounds than other approaches. This suggests that the approach of the fourth example can reduce the occurrence of false positive compounds and can more efficiently design compounds having higher selectivity. This proves that combining the selectivity prediction model with the evaluation function of the optimization method and using the combined selectivity prediction model together with the interaction prediction model provide effects of real time optimization of the structure in consideration of the selectivity.

REFERENCE SIGNS LIST

1   Compound Designing Device

2   Input Unit

3   Processing Unit

4   Memory Unit

5   Output Unit

**Claims**

1. A compound designing device comprising:

   an input unit configured to receive, at least about one or more query proteins, one or more pieces of query protein information corresponding to the one or more query proteins; and
   a processing unit configured to perform steps of

   (a) generating one or more pieces of compound information,
   (b) computing a score indicating interaction potential between a compound corresponding to the compound information and each of the one or more query proteins,
   (c) updating the compound information by an optimization method with reference to the score computed at step (b) such that the interaction potential increases, and
   (d) repeating steps (b) and (c) a plurality of times, wherein

   the score computed at step (b) is at least a score obtained by machine learning using, as teacher data, a first combination of protein information and compound information respectively corresponding to a protein and a compound causing first interaction therebetween.

2. The compound designing device of claim 1, wherein
   the machine learning is a support vector machine, in which in addition to the first combination, a second combination of protein information and compound information corresponding to a protein and a compound causing second interaction therebetween is used as teacher data, a separating plane separating the first combination from the second combination is obtained, and the score indicates a distance of a combination of compound information for which the score is to be computed and protein information for which the score is to be computed from the separating plane.

3. The compound designing device of claim 1 or 2, wherein
   the optimization method is one or more selected from the group consisting of swarm intelligence optimization, evolutionary computation, and particle swarm optimization.

4. The compound designing device of any one of claims 1-3, wherein
   the processing unit performs, after the step (c), step of (c1) selecting a piece of compound information corresponding to the compound from pieces of compound information approximating the compound information updated at step (c) and determining the selected piece of compound information to be the updated compound information.

5. The compound designing device of claim 4, further comprising:

   a memory unit, wherein
   the memory unit stores the updated compound information as a history,

   the processing unit performs, after the step (c1), steps of:

   (c2) referring to the history stored in the memory unit, and determining whether or not the selected piece of compound information is identical with the compound information in the history, and
   (c3) if the selected compound information is determined to be identical with the compound information in the history at step (c2), selecting another compound information and performs step (c2) again, and if the selected piece of compound information is not identical with the compound information in the history at step (c2), determining the selected piece of compound information to be the updated compound information.

6. The compound designing device of claims 1-5, wherein
   the compound information includes pieces of fragment information corresponding to fragments generated by cleaving a chemical structure of a compound based on a predetermined rule.

7. The compound designing device of claim 6, wherein
   the predetermined rule is a rule in which when a plurality of cleavage positions exist in the chemical structure of an identical compound, fragments are preferably generated based on possible combinations of the cleavage positions.

8.  The compound designing device of claim 6 or 7, wherein
    the compound information is expressed as a direct sum of vectors existing in a space in which one or more principal components resulting from a principal component analysis of the pieces of fragment information are assigned to an axis.

9.  The compound designing device of claim 6 or 7, wherein
    the optimization method is particle swarm optimization, the number of constitutional units of fragments of a compound to be designed is set, and the position X of a particle representing the compound information is given by Expression 1

[Expression 1]

$$X = \begin{pmatrix} x_{11} & \cdots & x_{1n} \\ \vdots & \ddots & \vdots \\ x_{m1} & \cdots & x_{mn} \end{pmatrix}$$

where m is the maximum number of elements of the fragments, and n is the number of constitutional units, and the velocity V of the particle is given by Expression 2

[Expression 2]

$$V = \begin{pmatrix} v_{11} & \cdots & v_{1n} \\ \vdots & \ddots & \vdots \\ v_{m1} & \cdots & v_{mn} \end{pmatrix}$$

where m is the maximum number of elements of the fragments, and n is the number of constitutional units.

10. The compound designing device, wherein
    the score computed at the step (b) is obtained by combining a score obtained by machine learning using, as teacher data, the first combination of the protein information and the compound information respectively corresponding to the protein and the compound causing the first interaction therebetween with one or more selected from the group consisting of a score obtained by activity value prediction, a score obtained by selectivity prediction, a score obtained by a docking calculation, a score obtained by synthesis possibility prediction, a score obtained by ADME-Tox prediction, a score obtained by physical property prediction, and a score obtained by prediction of binding free energy obtained by molecular dynamics method.

11. The compound designing method using a computer, the method comprising steps of:

    (A) inputting, at least about one or more query proteins, one or more pieces of query protein information corresponding to the one or more query proteins to an input unit of the computer;
    (B) generating one or more pieces of compound information;
    (C) computing a score indicating the interaction potential between a compound corresponding to the compound information and each of the one or more query proteins;

(D) updating the compound information by an optimization method with reference to the score computed at score computing step (C) such that the interaction potential increases, wherein

step (C) and step (D) are repeated a plurality of times, and further, the score computed at step (C) is obtained by at least machine learning using, as teacher data, a first combination of protein information and compound information respectively corresponding to a protein and a compound causing first interaction therebetween.

12. The compound designing method of claim 11, wherein

the machine learning is a support vector machine,

in addition to the first combination, a second combination of a piece of protein information and a piece of compound information respectively corresponding to a protein and a compound causing second interaction therebetween is used as teacher data,

separating plane separating the first combination and the second combination is obtained, and

the or each score represents a distance one or more combinations of the one or each piece of compound information for which the or each score is to be computed and the one or each piece of protein information for which the or each score is to be computed from the separating plane.

13. A computer program causing a computer to design a compound, the computer program allows the computer to execute steps of:

(i) receiving, about one or more query proteins, one or more pieces of query protein information corresponding to the one or more query proteins;

(ii) generating one or more pieces of compound information;

(iii) computing a score indicative of interaction potential between a compound corresponding to the compound information and each of the one or more query proteins;

(iv) updating the compound information by an optimization method with reference to the score computed at step (iii) so that the interaction potential increases;

(v) repeating step (iii) and step (iv) a plurality of times, wherein

the score computed at step (iii) is obtained by at least machine learning using, as teacher data, a first combination of protein information and compound information respectively corresponding to a protein and a compound causing first interaction therebetween.

14. The compound designing method of claim 13, wherein

the machine learning is a support vector machine,

in addition to the first combination, a second combination of a piece of protein information and a piece of compound information respectively corresponding to a protein and a compound causing second interaction therebetween is used as teacher data,

a separating plane separating the first combination and the second combination is obtained, and

the or each score represents a distance one or more combinations of the one or each piece of compound information for which the or each score is to be computed and the one or each piece of protein information for which the or each score is to be computed from the separating plane.

Figure 1

FRAME

UNIT          FRAGMENT

THE NUMBER
OF UNITS          TOPOLOGY

2

3

4

⋮          . . . . .

## Figure 2

## Figure 3

MEMORY UNIT

INTERACTION
LEARNING MODEL

COMPOUND
INFORMATION
UPDATE HISTORY

4

PROCESSING UNIT

COMPUTE SCORE REPRESENTING
INTERACTION POTENTIAL (B)

REACH THE DEFAULT
NUMBER OF TIMES

YES

NO

UPDATE COMPOUND
INFORMATION BY OPTIMIZATION
METHOD (C)

SELECT COMPOUND INFORMATION
APPROXIMATING UPDATED
COMPOUND INFORMATION (C1)

REFER COMPOUND INFORMATION
UPDATE HISTORY (C2)

YES    IS SELECTED COMPOUND
INFORMATION IDENTICAL WITH
COMPOUND IN HISTORY ?(C3)

NO

STORE COMPOUND
INFORMATION UPDATE HISTORY

3

**Figure 4**

**Figure 5**

## Figure 6

| β2AR | | A | B | C | D | E | F | G | H |
|---|---|---|---|---|---|---|---|---|---|
| | | | | | | R1 | | | |
| R2 | 1 | 5.2 | 6.0 | 11.0 | 13.7 | 207.9 | 59.6 | 9.0 | -- |
| | 2 | 3.2 | 5.1 | 28.1 | 20.7 | 148.9 | 70.3 | 12.2 | -- |
| | 3 | 1.2 | 3.3 | 122.3 | 185.4 | 269.2 | 44.0 | 42.1 | -- |
| | 4 | 3.4 | 5.1 | 22.8 | 120.4 | 148.1 | 52.1 | 16.2 | -- |
| | 5 | 2.7 | 18.4 | 120.4 | 126.5 | 104.7 | 18.7 | 22.8 | -- |
| | 6 | 1.7 | 7.3 | 84.7 | 56.9 | 54.9 | 24.9 | 1.8 | -- |
| | 7 | 4.4 | 19.2 | 92.5 | -- | 117.8 | 83.0 | 102.1 | 506.4 |
| | 8 | 2.9 | 5.2 | 13.7 | 101.6 | 100.0 | 72.2 | 18.5 | -- |
| | 9 | 3.3 | 22.2 | 59.8 | -- | -- | 55.8 | 53.9 | -- |
| | 10 | 2.8 | 9.0 | 41.3 | -- | 974.8 | 60.4 | 35.3 | -- |
| | 11 | 7.5 | 31.6 | -- | -- | -- | -- | 81.6 | -- |
| | 12 | 6.6 | 17.7 | 37.7 | -- | -- | 64.1 | 26.9 | -- |
| | 13 | 4.2 | 22.9 | 148.9 | 219.9 | 66.8 | 43.2 | 36.2 | -- |

RESULTS OF
SECONDARY ASSAY    (IC50 ; μM)

■ <10 μM
■ 10~30 μM
■ 30~100 μM
□ 100~150 μM
□ >150 μM
-- UNDETECTED

**Figure 7**

## CDK2 QSAR model

calculated pIC50

## V1b QSAR model

calculated pIC50

**Figure 8**

## CDK2

| IC50 | QSAR | | | CGBVS | | | CGBVS×QSAR | | |
|---|---|---|---|---|---|---|---|---|---|
| | Precision | Recall | F-measure | Precision | Recall | F-measure | Precision | Recall | F-measure |
| 1000nM~ 10000nM | 1.82E-04 | 3.08E-01 | 3.64E-04 | 7.83E-04 | 6.08E-01 | 1.56E-03 | 2.60E-03 | 3.92E-01 | 5.16E-03 |
| 100nM~ 1000nM | 2.60E-04 | 3.97E-01 | 5.20E-04 | 1.13E-03 | 6.55E-01 | 2.26E-03 | 1.41E-03 | 4.20E-01 | 2.80E-03 |
| 10nM~100nM | 2.57E-04 | 4.06E-01 | 5.14E-04 | 8.43E-04 | 6.16E-01 | 1.68E-03 | 1.27E-03 | 4.64E-01 | 2.53E-03 |
| 1nM~10nM | 9.14E-05 | 2.38E-01 | 1.83E-04 | 3.27E-04 | 7.86E-01 | 6.54E-04 | 4.61E-04 | 2.14E-01 | 9.20E-04 |
| LESS THAN 1nM | 0.00E+00 | 0.00E+00 | 0.00E+00 | 0.00E+00 | 0.00E+00 | 0.00E+00 | 0.00E+00 | 0.00E+00 | 0.00E+00 |

## V1b

| IC50 | QSAR | | | CGBVS | | | CGBVS×QSAR | | |
|---|---|---|---|---|---|---|---|---|---|
| | Precision | Recall | F-measure | Precision | Recall | F-measure | Precision | Recall | F-measure |
| 1000nM~ 10000nM | 7.81E-05 | 1.48E-01 | 1.56E-04 | 3.21E-04 | 7.39E-01 | 6.42E-04 | 4.46E-04 | 1.48E-01 | 8.90E-04 |
| 100nM~ 1000nM | 9.84E-05 | 2.96E-01 | 1.97E-04 | 2.62E-04 | 6.54E-01 | 5.23E-04 | 7.12E-04 | 4.32E-01 | 1.42E-03 |
| 10nM~100nM | 2.80E-05 | 1.84E-01 | 5.59E-05 | 1.48E-04 | 7.89E-01 | 2.96E-04 | 2.46E-04 | 3.16E-01 | 4.91E-04 |
| 1nM~10nM | 1.55E-05 | 1.50E-01 | 3.10E-05 | 7.90E-05 | 8.00E-01 | 1.58E-04 | 2.23E-04 | 3.50E-01 | 4.46E-04 |
| LESS THAN 1nM | 1.75E-05 | 6.67E-01 | 3.51E-05 | 2.96E-05 | 1.00E+00 | 5.93E-05 | 2.15E-04 | 6.67E-01 | 4.30E-04 |

## INTERNATIONAL SEARCH REPORT

International application No.

PCT/JP2013/072630

| A. CLASSIFICATION OF SUBJECT MATTER |
|---|
| *G06F19/16*(2011.01)i, *C07B61/00*(2006.01)i, *C40B30/02*(2006.01)i, *G01N33/15* (2006.01)i, *G01N33/50*(2006.01)i |

According to International Patent Classification (IPC) or to both national classification and IPC

| B. FIELDS SEARCHED |
|---|
| Minimum documentation searched (classification system followed by classification symbols) |
| G06F19/16, C07B61/00, C40B30/02, G01N33/15, G01N33/50 |

| Documentation searched other than minimum documentation to the extent that such documents are included in the fields searched |
|---|
| Jitsuyo Shinan Koho        1922-1996    Jitsuyo Shinan Toroku Koho    1996-2013 |
| Kokai Jitsuyo Shinan Koho  1971-2013    Toroku Jitsuyo Shinan Koho    1994-2013 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | Hassen Mohammed Alsafi, et al., Rational Drug Design using Genetic Algorithm : Case of Malaria Disease, Journal of Emerging Trends in Computing and Information Sciences, 2012.07, VOL. 3, NO. 7, p.1093-1102 | 1-14 |
| Y | Fabian Dey, et al., Fragment-Based de Novo Ligand Design by Multiobjective Evolutionary Optimization, J. Chem. Inf. Model, 2008.02.29, Vol.48, No.3, p.679-690 | 1-14 |
| Y | WO 2007/139037 A1 (Kyoto University), 06 December 2007 (06.12.2007), paragraphs [0036] to [0235] & US 2010/0099891 A1   & EP 2031528 A1 & WO 2007/139037 A1 | 1-14 |

| [X] Further documents are listed in the continuation of Box C. | [ ] See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered    to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 24 October, 2013 (24.10.13) | 05 November, 2013 (05.11.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2013/072630 |

C (Continuation).　DOCUMENTS CONSIDERED TO BE RELEVANT

| Category* | Citation of document, with indication, where appropriate, of the relevant passages | Relevant to claim No. |
| --- | --- | --- |
| Y | IQBAL Mudassar, et al., Protein Interaction Inference Using Particle Swarm Optimization Algorithm, Lect Notes Comput Sci, 2008, Vol.4973, p.61-70 | 9 |
| A | WO 2008/53924 A1  (Keio University), 08 May 2008 (08.05.2008), paragraphs [0028] to [0047] & US 2010/0070438 A1　　& EP 2083265 A1 & WO 2008/053924 A1 | 1-14 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009007302 A
- JP 2008217594 A
- JP 2008081435 A

- WO 2007139037 A
- WO 2008053924 A

**Non-patent literature cited in the description**

- **HARTENFELLER, M. ; SCHNEIDER G. et al.** Concept of combinatorial de novo design of drug-like molecules by particle swarm optimization. *Chemicalbiology & drug design,* 2008, vol. 72, 16-26